# EUROPEAN PATENT SPECIFICATION

(11) **EP 1 774 936 B1**
(45) Date of publication and mention of the grant of the patent: **29.06.2011**
(21) Application number: 05762020.5
(22) Date of filing: 21.07.2005
(51) Int. Cl.: A61F 13/15

(54) **SHORTS TYPE DISPOSAL DIAPER**
EINWEG-WINDEL VOM HÖSCHENTYP
COUCHE JETABLE DE TYPE CALECON

(30) Priority: 30.07.2004 JP 2004224888; 30.07.2004 JP 2004224891
(43) Date of publication of application: 18.04.2007
(73) Proprietor: KAO CORPORATION, Tokyo 103-8210 (JP)
(72) Inventor: KANAI, Taeko, c/o Kao Corporation, Res. Laboratories, Tochigi 321-3497 (JP); SASAKI, Jun, c/o Kao Corporation, Res. Laboratories, Tochigi 321-3497 (JP); OTSUKA, Michiko, c/o Japan Women's University, Tokyo 112-8681 (JP)
(74) Representative: Vossius & Partner
(86) International application number: PCT/JP2005/013356
(87) International publication number: WO 2006/011407

(56) References cited:
- JP-A- 5 192 366
- JP-A- 57 117 602
- JP-A- 62 243 806
- JP-A- 2001 178 770
- JP-A- 2002 159 529
- JP-U- 2 084 623
- JP-U- 7 043 075
- US-A- 5 853 402
- US-B1- 6 503 239

## Description

### Technical Field

The present invention relates to a pull-on disposable diaper, especially the one designed for infants.

### Background Art

Conventional pull-on disposable diapers tend to puff out and become baggy around the wearer's crotch while worn. Baggy diapers interfere with the leg movement of the wearer and often get out of right position depending on the wearer's movement. If a diaper gets out of position, a leak can follow. Additionally, bagginess of a diaper makes it difficult to put outer clothing, such as shorts and pants, over the diaper only to provide a droopy appearance. As a diaper gets out of position due to the bagginess around the crotch, the diaper becomes baggier.

To address the problem, various techniques relating to narrowing the crotch portion of a diaper have been proposed as in JP-A-2002-159529, JP-A-U-2-84623, and JP-A-11-188056. However, a narrowed crotch portion looks like a sagging belt while worn only to result in more bagginess. No improvements are obtained on freedom of wearer's movement or fit of outer clothing such as shorts and pants. No improvements on diaper appearance during use are obtained, either. The diaper disclosed in JP-A-2002-159529, in particular, has not only a narrowed crotch portion but also a shortened length of side seals relative to the total diaper length, that is, an increased length of the non-sealed portion (namely, the crotch portion). Such a design helps the crotch portion droop further like a sagging belt.

Apart from narrowing the crotch portion, techniques for preventing a diaper from being displaced away from its right position have been proposed. For example, JP-A-9-84826 proposes a pull-on disposable diaper, at least the stomach side of which has a first portion within an area 20 mm above the front end of the absorbent core and 20 mm below the front end of the absorbent core and a second portion defined between the first portion and the leg openings. Elastic members are spacedly disposed in the first portion at a smaller interval than in the second portion. The proposal aims at leak prevention without impairing the wearing comfort by making the contact with the wearer's body closer in the first portion than in the second one while substantially equalizing the planar pressure per given area between the first and the second portions.

A pull-on disposable diaper having elastic members disposed at an interval gradually decreasing toward the waist openings is known from -JP-A-U-6421. This configuration allows for a strong contractive force being applied on the upper part of the wearer's hipbone.

However, the above-described diaper configurations are not sufficiently contoured to the anatomy of a wearer's body and are still liable to slide down while worn to damage the appearance or interfere with the wearer's movement. Moreover, the strong constrictive force of the waist portion can make diapering difficult.

A leg hole of a pull-on disposable diaper is generally defined by an arc-shaped curve. Unlike that, a diaper whose leg hole is defined by a curve gouged inwardly in the front side of the diaper as disclosed, e.g., in JP-A-9-66071 and WO96/40035. The aim of so shaping the leg hole is not to provide a fit of the leg holes to the wearer's groins, so that the shape of the leg holes cannot be seen as optimized to the body shape of a wearer. Therefore, where an elastic member is disposed around the leg holes to avoid gapping around the wearer's legs, too much constriction tends to be exerted. Furthermore, the diaper is apt to slide down with the wearer's movement and cannot be seen as looking neat during wear.

Further related art is shown in US-B1-6 503 239 and US-A-5853402.

### Disclosure of the Invention

The present invention provides a pull-on disposable diaper according to claims 1 to 7.

### Brief Description of the Drawings

Fig. 1 is a perspective of an embodiment of a pull-on disposable diaper according to the present invention.
Fig. 2 is an exploded perspective of the diaper of Fig. 1 before assembly.
Fig. 3 is a plan of an exterior material of Fig. 2 in its flat-out state.

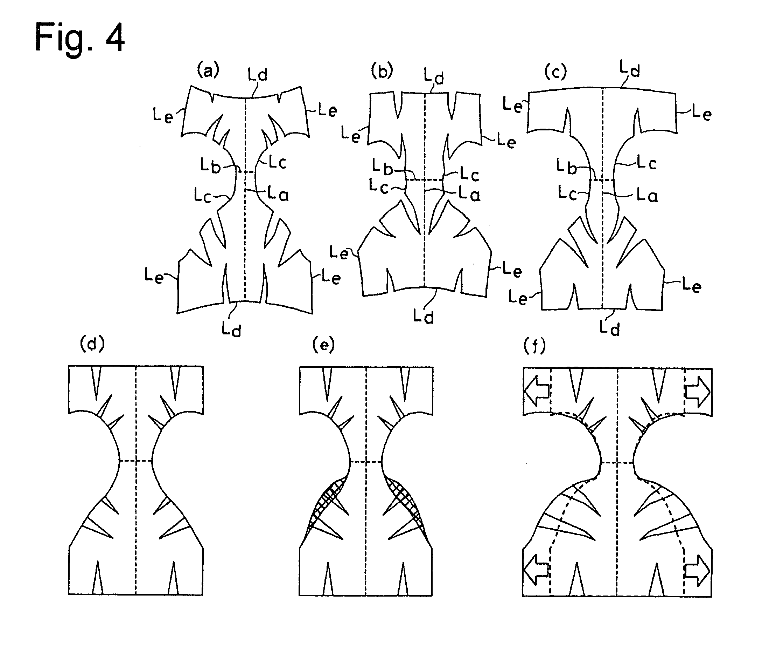

Fig. 4(a), Fig. 4(b), Fig. 4(c), Fig. 4(d), Fig. 4(e), and Fig. 4(f) represent a novel diaper design approach, in which Fig. 4(a), Fig. 4(b), and Fig. 4(c) each illustrate a base pattern obtained by draping, Fig. 4(d) illustrates a revised base pattern obtained by adding correction to the base pattern, Fig. 4(e) is a revised master pattern obtained by adding correction to a master pattern taking extension and contraction of the wearer's skin into consideration, and Fig. 4(f) is a final pattern obtained by adding correction to the revised master pattern taking ease of diapering into consideration.
Fig. 5 illustrates body measurements needed in the novel diaper designing.
Fig. 6 is a plan showing the structure of an absorbent core.
Fig. 7 is an illustration of the ilia.
Fig. 8 illustrates how to measure the front-to-rear length at the height of the anterior superior iliac spine.
Fig. 9(a) is a perspective of another embodiment of the diaper according to the first aspect of the present invention, and Fig. 9(b) is a plan of the diaper of Fig. 9(a) in its flat-out state.
Fig. 10(a), Fig. 10(b), and Fig. 10(c) each illustrate a plan of another embodiment of the absorbent core.
Fig. 11(a) and Fig. 11(b) are each a plan of still another embodiment of the absorbent core.
Fig. 12(a), Fig. 12(b), Fig. 12(c), Fig. 12(d), and Fig. 12(e) are each a plan of yet another embodiment of the absorbent core.
Fig. 13 is a plan of an exterior material used in a pull-on disposable diaper according to an embodiment of the present invention in its flat-out state.
Fig. 14 illustrates a fragmentary plan of another embodiment of a diaper according to the present invention.

### Detailed Description of the Invention

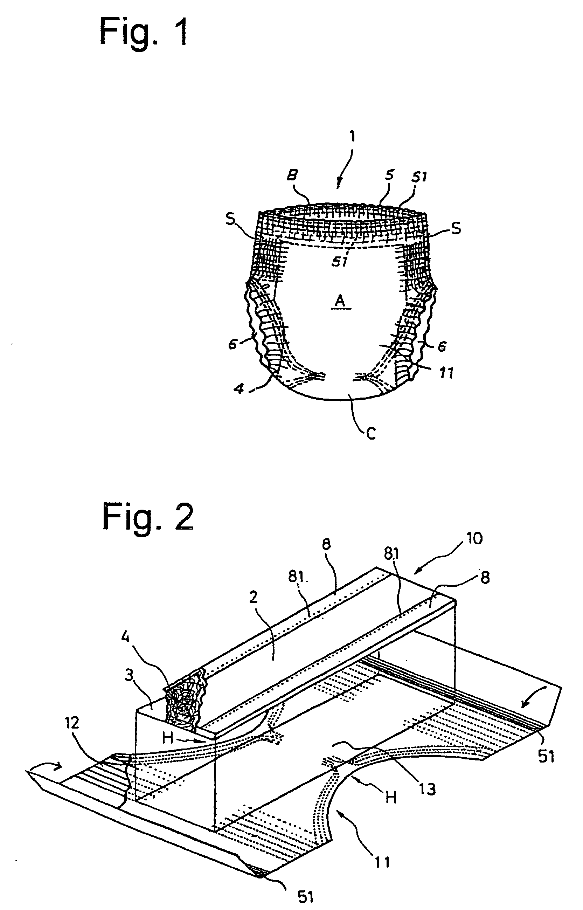

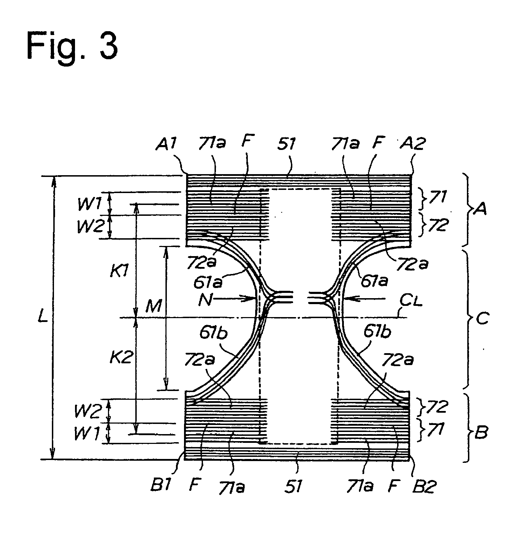

The present invention will be described based on its preferred embodiments with reference to the accompanying drawings. Fig. 1 is a perspective view of an embodiment of the pull-on disposable diaper according to the invention. Fig. 2 is an exploded perspective view illustrating the diaper of Fig. 1 before assembly. Fig. 3 is a plan of the diaper shown in Fig. 2 in its flat-out state. The term "flat-out state" denotes the state before the lateral side edges of a diaper are connected together.

To begin with, the novel design approach that provided the foundation for the completion of the present invention is briefly described taking an example.

### Step 1:

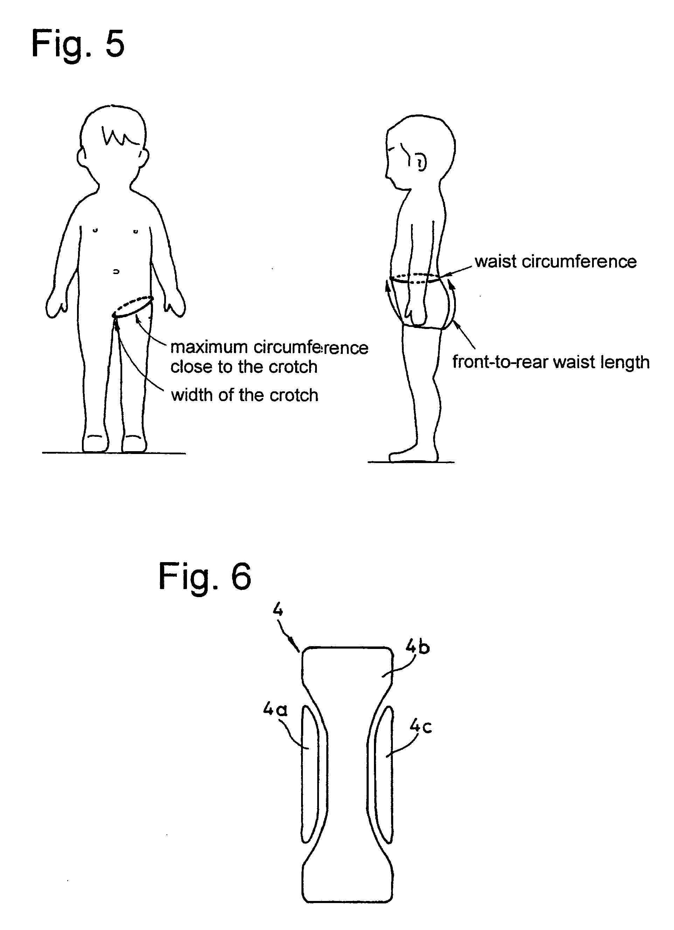

Sheeting broad enough compared with a flat out contour of an absorbent article is directly applied to the body of a wearer (e.g., an infant) to do draping. The sheeting on the body is marked with lines indicating the waist, the thigh circumference (the maximum circumference close to the crotch, see Fig. 5), and the side seam and the folded darts. Accurate measurements are taken on the right side of the body. The sheeting is unfolded, and the resulting pattern of the right side is scanned into a CAD program, copied to make a horizontal flip image, which is taken as a base pattern for planar design. Examples of base patterns thus prepared are shown in Figs. 4(a), 4(b), and 4(c). In these figures, vertical line segment La indicates the length along the body from the middle of the front waistline to the middle of the back waistline via the crotch (hereinafter "front-to-rear waist length")(see Fig. 5); horizontal line segment Lb that is perpendicular to the vertical segment La indicates the crotch position (the position in the longitudinal direction at which the distance between the left and the right thigh circumferential lines is the least, corresponding to the position in the longitudinal direction at which the crotch of the wearer is narrowest) and the crotch width: curve Lc indicates the thigh circumferential line; curve Ld represents the waistline; and curve Le is the side seam. In this way, a base pattern is taken from a plurality of wearers.

### Step 2:

The prepared base patterns are divided into groups according to age (of months), sex, etc. and averaged per group to prepare a master pattern for each group. Measurements of waist, front-to-rear waist length, side seam, and thigh circumference were taken on about 350 babies and toddlers to obtain the averages for each group, which were used in the master pattern making. The shape of the thigh circumferential line, the crotch position and width, and the position, the number, and the size of the darts were averaged per group. Fig. 4(d) illustrates a master pattern obtained in that manner. All the available base patterns may be averaged to make a sole master pattern.

### Step 3:

Wearers are allowed to move freely, and their behavior was recorded on videotape, etc. The body movements frequently shown and how the wearer's skin extends and contracts with such movements are observed. As a result of observation and analysis on the children's behavior, it has been revealed that children of age who wear a pull-on absorbent article (particularly pull-on diaper) frequently show actions such as crawling, movement from standing to sitting or vice versa, bending forward while sitting, and running about and that these actions are accompanied by frequent extension and contraction of the skin on the back of the thighs

### Step 4:

To each of the master patterns (or the single master pattern) obtained in step 2 is added correction reflecting the findings about the skin extension and contraction ascertained in step 3. The correction is made for the purpose of securing a region that can follow the skin extension and contraction. Fig. 4(e) illustrates a revised master pattern obtained by adding a cross-hatched region to the master pattern in order to afford to an absorbent article a region that follows extension and contraction of the skin in the back of a wearer's thighs.

### Step 5:

To the revised master pattern is added correction for imparting ease of putting on (ease of diapering) a wearer. Fig. 4(f) shows a pattern obtained by enlarging the revised master pattern in the width direction at a prescribed ratio. The result of that correction is the final pattern furnishing the planar shape of a diaper. Taken directly from the body contour of children, the thus obtained pattern is highly body-fitted.

The diaper 1 according to the present embodiment is composed of a substantially rectangular absorbent body 10 and an exterior material 11. The absorbent body 10 is composed of a liquid permeable topsheet 2, a liquid impermeable or water repellent backsheet 3, and a liquid retentive absorbent core 4 interposed between the two sheets 2 and 3. The exterior material 11 is disposed on the side of the backsheet 3 of the absorbent body 10.

The exterior material 11 has its longitudinal central portion narrowed in a sandglass shape to define the outline of the diaper. The exterior material 11 forms a leg hole H on both sides thereof (see Fig. 2). The exterior material 11 is sectioned, in the longitudinal direction, into a stomach portion A that is to be located on the wearer's stomach side, a back portion B that is to be located on the wearer's back side, and a crotch portion C between the portions A and B. The stomach portion A and the back portion B correspond to the front portion and the rear portion, respectively in the longitudinal direction of the exterior material 11, and the crotch portion C corresponds to the lengthwise central portion of the exterior material 11. As shown in Fig. 3, the exterior material 11 has a flap F extending transversely of both the front portion and the rear portion. The lateral side edges A1 and A2 of the flaps F in the stomach portion A and the lateral side edges B 1 and B2 of the flaps F in the back portion B are connected together to form a waist opening 5 and a pair of leg openings 6 in the diaper 1 shown in Fig. 1. A pair of side seals S are formed on both side seams of the diaper 1 by this connection. Connection can be carried out by, for example, heat sealing, high frequency sealing or ultrasonic sealing.

The topsheet 2 and the backsheet 3 are each rectangular and united together with the absorbent core 4 to form an oblong rectangular absorbent body 10. The topsheet 2 and the backsheet 3 can be of materials commonly used in this type of diapers. The details of the absorbent core 4 will be given later.

As shown in Fig. 2, a side cuff 8, 8 made of a liquid impermeable or water repellent, breathable material is provided on each long side of the absorbent body 10. The side cuff 8 has a fixed edge and a free edge along the length direction of the absorbent body 10. The fixed edge is fixed to the topsheet 2. A side cuff elastic member 81 is disposed in its stretched state along and near the free edge, whereby the pair of the side cuffs 8 stand to block the flow of liquid in the width direction of the absorbent body 10.

The exterior material 11 has at least two sheets of nonwoven fabric, i.e., an outer nonwoven sheet 12 serving as the outer surface of the diaper 1 (the side opposite to a wearer's side) and an inner nonwoven sheet 13 located on the inner side of the outer nonwoven sheet 12. The inner nonwoven sheet 13 is joined to the inner side of the outer nonwoven sheet 12 with an adhesive, such as a hot-melt adhesive. It is preferred for both the outer nonwoven sheet 12 and the inner nonwoven sheet 13 to be made of water repellent nonwoven fabric so as to prevent liquid oozing from the outside and the inside of the diaper.

The exterior material 11 extends outward from both longitudinal ends of the absorbent body 10, and the extension is folded back to cover each of the longitudinal ends of the absorbent body 10 (specifically, the longitudinal ends of the topsheet 2 are covered with the extensions).

A plurality of waist elastic members 51, 51 are disposed along, and over the whole width of, the front and the rear ends of the exterior material 11 between the outer nonwoven sheet 12 and the inner nonwoven sheet 13 in their stretched state. The waist elastic members 51, 51 are arranged such that those on the stomach portion A and those on the back portion B overlap with each other at their ends when the side edges A1 and A2 of the stomach portion A and the side edges B 1 and B2 of the back portion B meet and are sealed together. As a result, there is formed a substantially continuous loop of waist gather along the waist opening 5 of the diaper 1 as shown in Fig. 1.

Leg elastic members 61a and 61b are disposed in the curved portions on both sides of the exterior material 11. Each of the leg elastic members 61a and 61b is along the curve. The leg elastic members 61a and 61b are sandwiched between the outer nonwoven sheet 12 and the inner nonwoven sheet 13 and fixed in their stretched state by prescribed fixing means. The leg elastic member 61a and the leg elastic member 61b overlap at one of their ends in the crotch portion C. The other end of each of the leg elastic members 61a and 61b is located at the side edge A1 or A2 of the stomach portion A and the side edge B 1 or B2 of the back portion B, respectively. The leg elastic members 61 a and 61 b are arranged such that their ends located at the side edges A1 or A2 of the stomach portion A and their ends located at the side edge B 1 or B2 of the back portion B overlap with each other when the side edges A1 and A2 of the stomach portion A and the side edges B1 and B2 of the back portion B are joined together. As a result, there is formed a substantially continuous loop of leg gather along each of the leg openings 6, 6 of the diaper 1 as shown in Fig. 1.

The diaper 1 has a number of elastic members extending in the width direction between the waist opening 5 and the leg openings 6 in each of the stomach portion A and the back portion B. By disposing the elastic members, a first portion 71 and a second portion 72 both extending transversely of the diaper 1 are formed between the waist opening 5 and the leg opening 6. First elastic members 71 a are disposed in the first portion 71, and second elastic members 72a are disposed in the second portion 72. The first portion 71 is positioned between the waist opening 5 and leg openings 6, and the second portion 72 is positioned between the first portion 71 and the leg openings.

All the first elastic members 71 a and the second elastic members 72a are fixed in their stretched state between the outer nonwoven sheet 12 and the inner nonwoven sheet 13. The first elastic members 71a are arranged such that their ends located at the side edges A1 or A2 of the stomach portion A and their ends located at the side edge B1 or B2 of the back portion B overlap with each other when the side edges A1 and A2 of the stomach portion A and the side edges B 1 and B2 of the back portion B are joined together. The second elastic members 72a are arranged in the same way. As a result, there is formed a gather in the first portion 71 and the second portion 72 in the stomach portion A as illustrated in Fig. 1 and also, while not shown, in the back portion B.

Each of the first elastic members 71 a and the second elastic members 72a extends between the lateral sides of the diaper 1 (i.e., the lateral side edges of the exterior material 11) and the lateral, long side edges of the absorbent core 4. Substantially neither the first elastic members 71 a nor the second elastic members 72a exists in the area where the absorbent core 4 exists. That is, the gathers formed in the first portion 71 and the second portion 72 are located between the lateral sides of the diaper 1 and the lateral side edges of the absorbent core 4. There is substantially no gathers in the area where the absorbent core 4 is disposed. Therefore, contraction of the exterior material 11 due to contraction of the first elastic members 71a and the second elastic members 72a does not occur in the area where the absorbent core 4 exists, so that the diaper 1 not only keeps its neat appearance but exhibits satisfactory absorption capacity. Nevertheless the present invention is not limited to that configuration. The first elastic members 71a and the second elastic members 72a may be disposed substantially over the whole circumference of the diaper 1.

The elastic members used in the diaper 1 according to the present embodiment preferably include natural rubber, polyurethane resins, foamed urethane resins, extensible nonwoven fabrics, and hot-melt extensible materials molded into a string, a tape, a net or film.

According to the present embodiment, as illustrated in Fig. 3, the distance M between the lower end of the flap F in the longitudinally front portion and the lower end of the flap F in the longitudinally rear portion of the diaper 1 is 210 to 280 mm measured in the flat state of the diaper 1. The distance M defined above will hereinafter be referred to as "flap-to-flap distance". The flap-to-flap distance M is the flap-to-flap distance of the final pattern obtained by the above-described design approach with a necessary length added taking the thickness of diaper materials into consideration. The flap-to-flap distance M is a length optimized to the wearer's figure, characterized by being shorter than the flap-to-flap distance in conventional pull-on diapers. The flap-to-flap distance M is a measure of the length or area of the portion located in the crotch area of a wearer. In detail, a pull-on diaper having a longer flap-to-flap distance M has a longer length or a larger area in the portion located in the crotch area of a wearer, and vice versa. As a result of optimization to the wearer's figure, the diaper 1 of the present embodiment has a shorter flap-to-flap distance M than conventional pull-on diapers, that is, the portion located in the crotch area of a wearer is shorter in length or smaller in area than before. It follows that the portion located in the crotch area of a wearer has a reduced proportion in the absorbent core 4 of the diaper 1 so that bagginess of the crotch portion C of the diaper 1 is reduced to give a neat appearance as a whole diaper. The wearer's movement, particularly the leg movement is not hindered. The diaper hardly slides down with the wearer's movement while worn so that urine and fecal leakage hardly occurs. Outer clothing such as a shorts or a pants can easily be put over the diaper to give a neat appearance.

To further improve the appearance of the crotch portion C of the diaper 1, the flap-to-flap distance M is preferably 230 to 270 mm, more preferably 230 to 260 mm.

The whole length L of the exterior material 11 in its flat state is selected arbitrarily. Nevertheless, L is preferably 460 to 520 mm taking into consideration a neat appearance during and after use and prevention of sliding down. For assuring a sense of security, it is desirable for the diaper to completely cover the navel. From this viewpoint, L is more preferably 490 to 580 mm.

In order to improve the appearance around the crotch portion C by placing a proper amount (a proper length or area) of the absorbent core 4 on the wearer's crotch, not only the flap-to-flap distance M but the size of the crotch portion C in the diaper width direction is important. If the size of the crotch portion C is too big, it would not have a good fit to the wearer's crotch and become baggy. From this viewpoint, in the present embodiment, the width at the narrowest part of the crotch portion C, i.e., the width N at the narrowest part in the longitudinal central portion of the exterior material 11 in its flat state (see Fig. 3) is 50 to 160 mm. Where the narrowest part is not identified as a single position but extends in the length direction with the same width, the position at the center of the length of the part with the same width is defined to be the narrowest part.

The width N of the narrowest part is characterized by being smaller than that of conventional pull-on diapers. In short, in the diaper 1 of the present embodiment, the portion of the absorbent core 4 located in the wearer's crotch has a smaller size in both the length direction and the width direction than conventional pull-on diapers. To further improve the appearance of the crotch portion C, the width N at the narrowest part is preferably 60 to 140 mm, more preferably 60 to 120 mm.

As previously stated, in the diaper 1 of the present embodiment, the portion of the absorbent core 4 which is located in the wearer's crotch is reduced in both the length and the width directions. In other words, the amount of the portion of the absorbent core 4 located in the wearer's crotch is reduced, which may lead to a reduced absorption capacity of the diaper 1. To avoid this, the absorbent core 4 is configured to separate apart from the diaper main body (the exterior material 11 in the case of the present embodiment) in side parts located near the longitudinal side edges while having a part which is located between the side parts and is fixed to the diaper main body. More specifically, the absorbent core 4 has the following design of configuration.

As illustrated in Fig. 6, the absorbent core 4 used in the diaper 1 of the present embodiment is divided into three pieces, 4a, 4b, and 4c, each extending in the longitudinal direction and made of an airlaid mixture of fluff pulp and a superabsorbent polymer. The three pieces are wrapped all together in tissue paper (not shown) to constitute a unitary absorbent core 4. Division between the central piece 4b and the side pieces 4a and 4c is along the longitudinal middle portion of the absorbent core 4. The longitudinal front and rear portions of the absorbent core 4 are formed solely of the central piece 4b. The absorbent core 4 is adhesively fixed to the exterior material (not shown) on its lower side. The side pieces 4a and 4c are not fixed to the exterior material, being capable of separating from the exterior material. On the other hand, the rest of the divided pieces 4a and 4c, i.e., the central piece 4b is fixed to the exterior material 11. So configured, the side pieces 4a and 4c rise apart from the exterior material by the body pressure imposed in the width direction of the absorbent core 4 while the diaper 1 is worn. To ensure the rising properties of the side pieces, an elastic member is preferably disposed near the side edge of the side pieces 4a and 4c. As a result of the rising, the substantial width of the absorbent core 4 approaches the width of the central piece 4b. In short, although the absorbent core 4 has a large width before the diaper 1 is worn but has its width reduced to fit the wearer's crotch properly while the diaper 1 is worn. The pieces 4a and 4c on both sides assure a sufficient absorption capacity as a whole absorbent core 4. By this mechanism the diaper 1 of the present embodiment is prevented from reducing the absorption capacity despite of the reduced amount of the absorbent core 4 in the crotch of a wearer.

The substantial width of the absorbent core 4 during use (i:e., the width of the adhered area between the absorbent core 4 and the exterior material 11) is preferably set at 20 to 90 mm. In view of fit and bagginess prevention in the crotch portion C during use, that width is preferably 20 to 80 mm, more preferably 30 to 70 mm. The width of the absorbent core 4 before use (i.e., the total width of the absorbent core 4 before the side pieces 4a and 4c rise) is preferably 50 to 150 mm. To improve the absorption performance of the crotch portion C, that width is more preferably 60 to 150 mm, even more preferably 70 to 140 mm.

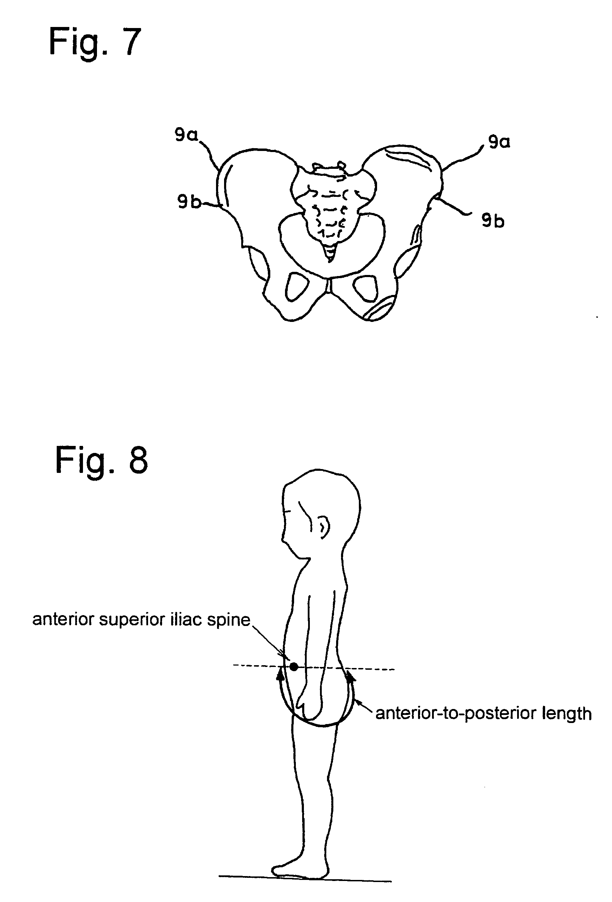

According to the present embodiment, the appearance of the diaper 1 while worn can further be improved by controlling the average pressure exerted on the wearer's body by the first portion 71 in a range of from 1.1 to 2.5 kPa, preferably 1.1 to 2.0 kPa, more preferably 1.2 to 1.8 kPa. The first portion 71 is preferably formed in the region of the diaper 1 which, while worn by a wearer, is applied to the part of the wearer's body between the left and the right iliac crests and the left and the right anterior superior iliac spines. That part of a wearer's body will sometimes be referred to the upper iliac region. "Iliac crest" and "anterior superior iliac spine", which are anatomical terms, mean the sites indicated by the reference numerals 9a and 9b, respectively, in Fig. 7. In order to prevent a pull-on diaper from sliding down, particularly to prevent a pull-on diaper worn by a child from sliding down or drooping at the waist, it has been considered effective to increase the constrictive pressure of the elastic members disposed in the waist portion thus bringing the pull-on diaper into close contact with a wearer's body as exemplified by the design of JP-A-U-2-84623 supra. On the contrary, as a result of investigation seeking for a solution of the problem that a pull-on diaper tends to slide down while worn, the present inventors have found it more effective to increase the constrictive pressure of the portion corresponding to the wearer's upper iliac region than the constrictive pressure of the waist portion. The reason is as follows. Because a diaper wearer, especially a child has a protruding abdomen as a physical characteristic, an increased constrictive pressure of the waist of a pull-on diaper applied around the periphery of the protruding belly gradually makes the waist of the diaper to constrict, thereby causing the diaper to droop until it fits the contour of the wearer.

The pressure exerted by the first portion 71 of the diaper 1 while worn (wearing pressure) is adjustable, for example, by controlling the material, thickness, elongation, and the distance of spacing of the first elastic members 71 a

Measurement of the wearing pressure of the first portion 71 of the diaper 1 is carried out on the diaper 1 put on a cylinder having a circumference of 500 mm with a clothing pressure measuring device (air-pack type contact surface pressure measuring system AMI 3037-2, available from AMI Techno Co., Ltd.) as follows.

### Measurement of pressure by first portion 71:

An air pack having a diameter of 15 mm is placed with its center even with the waist opening edge of the diaper, and the wearing pressure P1 is measured. The air pack setting position in the width direction of the diaper is nearly the center between the lateral side edges of the diaper and the lateral side edges of the absorbent core 4 (see Fig. 3). Subsequently, the air pack is shifted down by 5 mm along the diaper length direction to measure the wearing pressure (P2). The same measurement is repeated at 5 mm interval to obtain P3, P4, P5 ... , Pn). The measurements for obtaining P1 to Pn are made within an area between the waist opening and the leg openings where the both lateral sides are sealed together. The measurement is conducted at four points at every vertical position in each of the stomach portion A and the back portion B, two in the left side and two in the right side of the absorbent core 4, to obtain an average wearing pressure at a specific vertical position of the stomach portion A or the back portion B. The vertical distance between two out of the n sites of measurement between which all the measured pressures P are within a range of 1.1 kPa and 2.5 kPa is taken as the width of the first portion 71. When, for instance, P3 to P6 fall within the recited range, the width of the first portion 71 is (6-3)x5=15 mm. In that case, the mid point between the measuring site of P3 and that of P6 is the widthwise center of the first portion 71.

The mean circumference of the waist of children at which the diaper 1 of the present embodiment is primarily targeted is about 500 mm, which is selected as the diameter of the cylinder. The term "circumference of the waist" as used herein is an average of the circumference measured of a child in a standing posture and that of a child in a sitting posture, taking into consideration a probable change in the circumference at the waist with the change in body posture.

There is a certain distance (width) between the iliac crest and the anterior superior iliac spine of a wearer. The diaper 1 is effectively kept from moving down by applying the first portion 71 of the diaper 1 to an area within that width of the upper iliac region. From this point of view, the first portion 71 of the diaper 1 according to the present embodiment preferably has a width W1, measured in the diaper length direction, of 15 to 35 mm. With the width W1 being 20 to 35 mm, particularly 25 to 30 mm, the diaper 1 will be kept in place more effectively, and the appearance of the diaper 1 while worn and the ease of diapering with the diaper 1 will be further improved.

In order for the first portion 71 to be applied to the part between the iliac crest and the anterior superior iliac spine, the relation between the size of the diaper 1 and the physical size of a wearer is of importance. Considering children, primarily targeted wearers, for instance, the first portion 71 can successfully be applied to the upper iliac region of a wearer when the distance K1 from the widthwise center of the first portion 71 (i.e., the center of the first portion 71 along the diaper length direction) of the stomach portion A to the lateral centerline CL of the diaper 1 is preferably 180 to 220 mm as measured in the flat state of the diaper 1, and when the distance K2 from the widthwise center of the first portion 71 (i.e., the center of the first portion 71 along the diaper length direction) of the back portion B to the lateral centerline CL of the diaper 1 is preferably 180 to 220 mm as measured in the flat state of the diaper I . The K1 and K2 values have been decided as a result of body measurement of about 350 children for whom pull-on type diapers are primarily designed. These values will be described more specifically by referring to Fig. 8. The mid point between, and at the height of, the left and right anterior superior iliac spines in the anterior view of a child is designated "anterior center". The mid point similarly defined but in the posterior view of the child is designated "posterior center". The length from the anterior center via the crotch to the posterior center is designated "anterior-to-posterior length". The sum of the anterior-to-posterior length and an allowance for the thickness of the diaper materials is divided by two to give the above-recited K1 and K2 values. In order to apply the first portions 71 to the upper iliac region of a wearer more successfully, the distances K1 and K2 are more preferably 185 to 215 mm, even more preferably 190 to 215 mm.

The first portion exists in each of the stomach portion A and the back portion B. The wearing pressure of the first portion in the stomach portion A and that of the back portion B do not need to be quite the same. As long as the wearing pressure of each of the first portions 71 in the stomach portion A and the back portion B is in a range of from 1.1 to 2.5 kPa, the elastic members arranged in the stomach portion A and those in the back portion B of the diaper may differ in material, thickness, elongation, and distance of spacing. Nevertheless, excessive configurational difference between the stomach portion A and the back portion B can result in a dull appearance of the diaper 1 because the side seams of the exterior material 11 (where both the lateral sides of the front and those of the rear are connected) may come to the front or rear side of the diaper worn. Hence, it is desirable that the difference between a higher wearing pressure A of the first portion 71 of either the stomach portion A or the back portion B and a lower wearing pressure B of the first portion 71 of the opposite portion be such that the ratio (A-B)/A is within 30%.

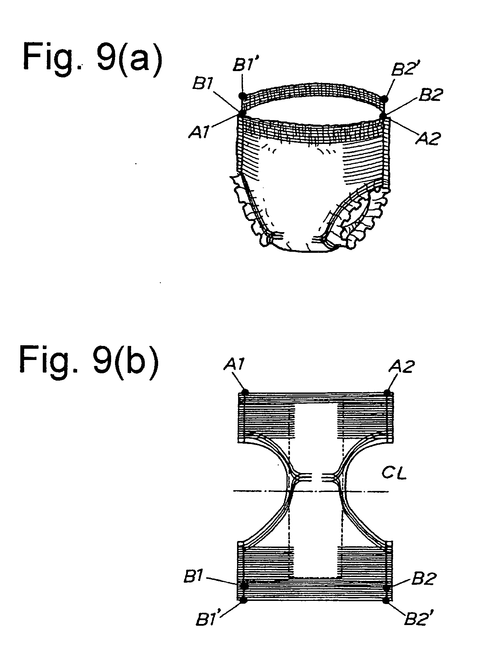

Where the side edges A1 and A2 of the stomach portion A and the side edges B1 and B2 of the back portion B are connected substantially in alignment with each other, the term "lateral centerline CL" as used in the present invention means the straight line parallel to the diaper width direction and passing the mid point between A1 and B1 in the flat state of the diaper 1 (see Fig. 3). Unlike that, there is a case where, as shown in Figs. 9(a) and 9(b), the side edges A1 and A2 of the stomach portion A are not connected with side edges B1' and B2' of the back portion B but with side edges B and B2 of which the upper ends are positioned lower than those of the side edges B1' and B2', respectively. In that case, the lateral centerline is drawn in the same manner as described above, except that the width defined by the upper ends of the side edges B1' and B2' and the upper ends of the side edges B1 and B2 is assumed not to exist.

It is preferred that the constrictive force of the diaper 1 according to the present embodiment rests primarily on the first elastic members 71a disposed in the first portions 71. In other words, it is not necessary to use the constrictive force of the elastic members disposed in the waist portion 5 as a primary means for keeping the diaper 1 in position on a wearer's body unlike the conventional pull-on diapers. On the contrary, an increased constrictive force of the waist portion 5 helps the diaper 1 move down as has been confirmed by the present inventors. From this point of view, in the diaper 1 of this embodiment, the average pressure imposed by the waist portion 5 during wear is preferably in the range of from 0.3 to 1.5 kPa, which range is lower than that of conventional pull-on diapers. It is preferred that the pressure of the waist portion 5 be lower than the average pressure of the first portion 71 by 0.5 to 1.0 kPa. The waist portion 5 having its constrictive pressure falling within the recited range offers another advantage that the waist portion 5 is easy to widen, which makes diapering easy. If the pressure of the waist portion 5 is less than 0.3 kPa, the natural length of the diaper 1 before being worn is so long that the diaper can have a poor appearance as a garment.

To ensure that the diaper is kept in place more effectively, the average pressure of the waist portion 5 is more preferably 0.4 to 1.2 kPa, even more preferably 0.4 to 1.0 kPa. The pressure of the waist portion 5 is measured in the same manner as used to measure the pressure of the first portions 71. That is, a 500 mm circumference cylinder is put in the diaper through the waist opening 5. An air pack of a clothing pressure measuring device is placed with its center positioned 15 mm below the waist opening edge to measure the wearing pressure. The measurement is conducted at 10 points at a 50 mm interval along the circumference. The average of the ten measurements is taken as a wearing pressure of the waist portion. Where the waist opening edge of the stomach portion and that of the back portion are not even as in the embodiment of Figs. 9(a) and 9(b), the position nearest to the waist opening edge in the overlap of the stomach and the back portions is taken as the waist opening edge. The pressure exerted by the waist portion 5 is adjustable by, for example, controlling the material, thickness, elongation, and the distance of spacing of the waist elastic members 51. Where the pressure by the waist portion 5 is in the range of the pressure by the first portion 71, the position of the measurement is included under the first portion 71.

The diaper 1 preferably has leg elastic members 61a and 61b arranged in their stretched state substantially along each of the leg openings. The average pressure exerted by each leg portion 6 while the diaper is worn preferably ranges from 1.0 to 2.4 kPa, more preferably 1.2 to 2.2 kPa. With the pressure by the leg portions 6 falling within that range during use, the opening edge of the leg portions 6 gives a snug fit to the groins to effectively prevent leakage around the wearer's leg. When the crotch portion of the diaper 1 gains weight due to discharge of urine or feces, the absorbent body in the crotch portion is kept in contact with the wearer's crotch by that pressure, thereby keeping the appearance of the diaper 1 and preventing leakage of urine and feces during wear. Thus, the pressure by the leg portions 6 is effective in maintaining the diaper 1 in good condition while worn.

The wearing pressure exerted by the leg portion is measured as follows with the same air-pack type contact surface pressure measuring system as used in the measurement of the pressure by the waist portion. A 300 mm circumference cylinder is inserted through the leg opening 6. The measurement is conducted in the area where the elastic member 61a or 61b is disposed at 6 points at a 50 mm interval along the circumference per leg portion 6. Where there are two or more elastic members 61 a or 61b, the point of measurement is the widthwise center of the area where the elastic members are arranged. The average of the 12 measurements (6 in the left leg portion and 6 in the right leg portion) is taken as a wearing pressure of the leg portion 6. Where there is no, or only partial, elastic members 61a, 61b around the leg openings, the wearing pressure of the area where the side cuff elastic member 81 is disposed measured in the part corresponding to each leg portion is taken as a pressure by the leg portion. The mean thigh circumference (at the largest point) of children at which the diaper 1 of the present embodiment is primarily targeted is about 300 mm, which is selected as the diameter of the cylinder.

The diaper 1 of the present embodiment preferably has an average wearing pressure of 0.2 to 0.8 kPa, more preferably 0.3 to 0.6 kPa, in the area except the first portions 71, the waist portion 5, and the leg portions 6, for example, the second portions 72 between the first portions 71 and the leg portions 6. With that wearing pressure, the diaper 1 is kept in close and comfortable contact with the wearer's body thus effectively preventing leakage. While the diaper 1 is worn, the second portions 72 are applied to the body below the upper iliac region, namely the lower abdominal region. The second portion 72 preferably has a width W2 (measured in the diaper length direction) of 40 to 70 mm, more preferably 45 to 65 mm.

Comparing the wearing pressure between different portions of the diaper 1, it is preferred that the first portion 71 be the highest, the waist portion 5 be the next highest, and the second portion 72 be the lowest. The diaper 1 with such an order of wearing pressure among the portions succeeds in keeping itself in place with a snug and comfortable fit to the wearer's body while worn thereby providing effective protection against leakage.

In the present invention, other configurations of the absorbent core 6 than that illustrated in Fig. 6 can be used. While in the embodiment shown in Fig. 6 the absorbent core 4 is divided into three pieces along the longitudinal direction thereof, the number of divided pieces is not limited to three and may be four or more as long as the pieces located on both lateral sides of the absorbent core 6 are configured to separate apart from the exterior material.

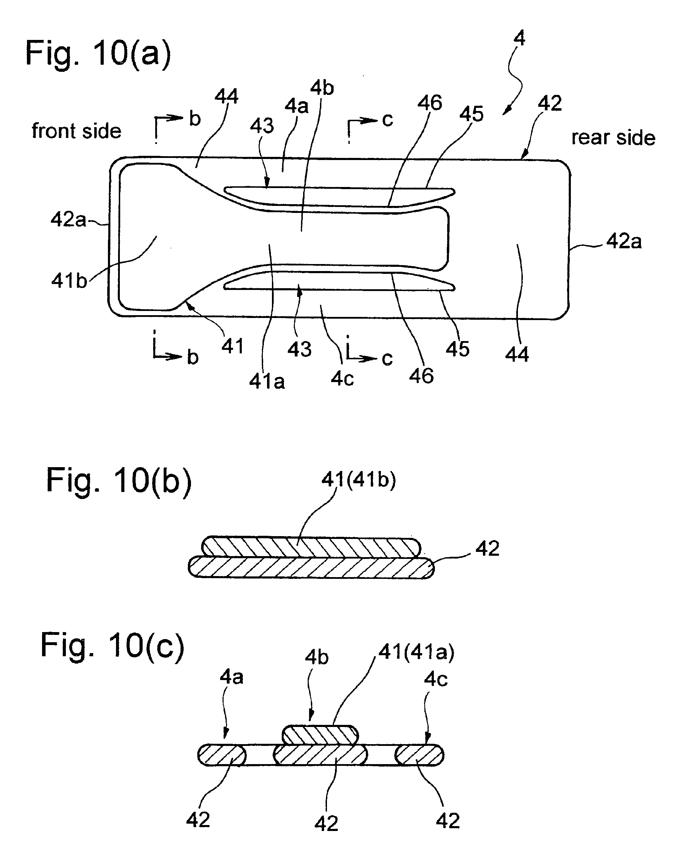

The shape of the absorbent core 4 is not limited to that of the aforementioned embodiment as long as the absorbent core 4 is capable of separating apart from the main body of the diaper near the both side edges thereof. For example, as illustrated in Fig. 10(a), the absorbent core 4 may have a double layer structure composed of a lower absorbent layer 42 and an upper absorbent layer 41 disposed on the lower absorbent layer 42. The upper absorbent layer 41 is nearly T-shaped. The lower absorbent layer 42 is larger in size than the upper absorbent layer 42 and rectangular, longer than wide. The lower absorbent layer 42 has a pair of defective parts 43 where the airlaid mixture is absent along the length direction. Each defective part 43 extends longitudinally of the lower absorbent layer 42. Each defective part 43 extends in the length direction only in the longitudinal central portion of the lower absorbent layer 42 without reaching the longitudinal ends 42a of the lower absorbent layer 42. Accordingly, the areas between the longitudinal ends of the lower absorbent layer 42 and the longitudinal ends of the defective parts 43 are airlaid parts 44, 44.

The two defective parts 43 are symmetric about the longitudinal centerline of the lower absorbent layer 42. The defective part 43 has the shape of a long bow, defined by a straight line segment 45 and an arc 46 connecting both ends of the segment 45. The straight line segment 45 is nearer to the side edge of the lower absorbent layer 42, with the arc 46 is farther from that side edge.

The nearly T-shaped upper absorbent layer 41 has a head 41 b and a leg 41 a. The leg 41a is positioned between the defective parts 43 of the lower absorbent layer 42. The leg 41a is almost as long as the defective parts 43. The head 41b is positioned on one of the airlaid parts 44 of the lower absorbent layer 42. The width of the head 41b is a little smaller than that of the lower absorbent layer 42. The side of the absorbent core 4 where the head 41b of the upper absorbent layer 41 is positioned is disposed in the back portion of the diaper.

In the above embodiment of the absorbent core 4, the parts of the lower absorbent layer 42 that are outboard of the defective parts 43 correspond to the side pieces 4a and 4c of the absorbent core shown in Fig. 6. The part of the lower absorbent layer 42 that is located between the defective parts 43 and the leg 41a of the upper absorbent layer 41 correspond to the central piece 4b of the absorbent core shown in Fig. 6. In the embodiment of Fig. 10, the parts outboard of the defective parts 43 have satisfactory rising capabilities because of the bow-like shape of the defective parts 43.

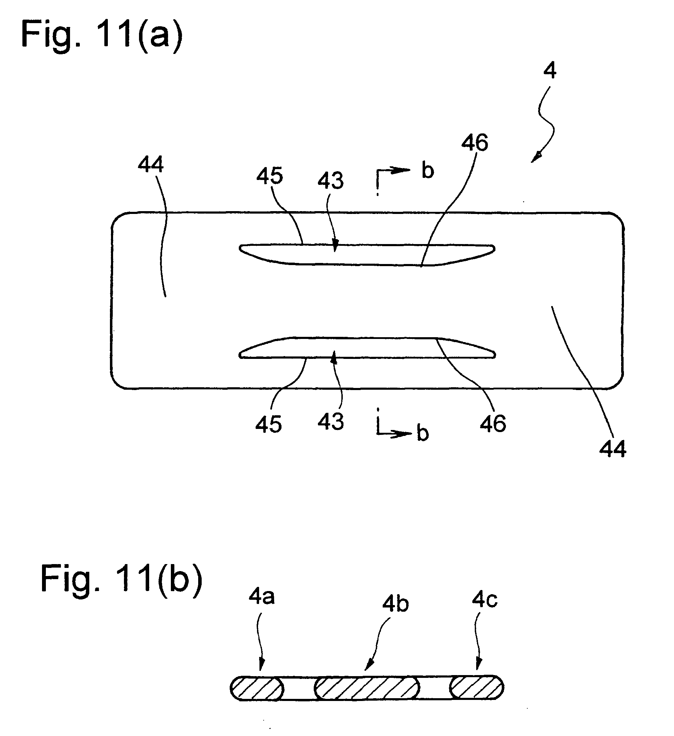

As a modification of the absorbent core of the embodiment illustrated in Figs. 10(a) through 10(c), the absorbent core 4 illustrated in Figs. 11(a) and 11(b) can be used. The absorbent core 4 of the embodiment shown in Figs. 11(a) and 11(b) consists solely of the lower absorbent layer used in the absorbent core of the embodiment of Figs. 10(a) to 10(c). In this modification, too, since there is a pair of defective parts 43 having the shape of a long bow formed along the length direction of the absorbent core 4, the parts outboard of the defective parts 43 exhibit satisfactory rising capabilities.

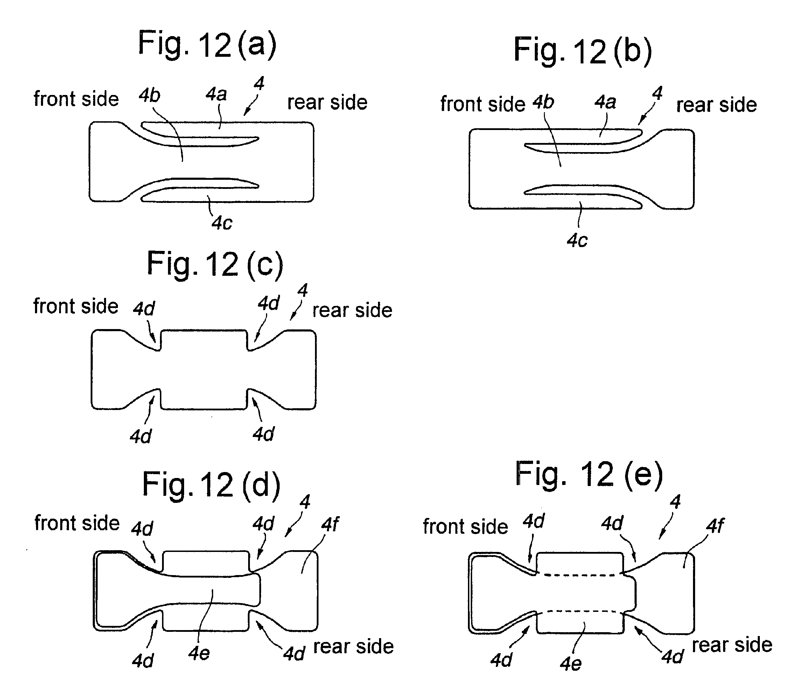

In Figs. 12(a) and 12(b) is illustrated an absorbent core 4 according to a modification of the absorbent core shown in Figs. 11(a) and 11(b), in which the defective parts 43 do not have a closed shape, and side pieces 4a and 4c extending in the length direction connect to the central piece 4b at their one end. In the embodiment shown in Fig. 12(c), a rectangular absorbent core 4 has notches 4d cut on each longer side edge thereof so that the part of the absorbent core 4 that is located between notches on each side may separate apart from the diaper main body. In the embodiments shown in Figs. 12(d) and 12(e), the absorbent core 4 has a double layer structure consisting of the upper layer 4e and a lower layer 4f. The upper layer 4e and the lower layer 4f are configured such that, when superposed on each other, the absorbent core 4 has notches 4d cut on each side edge thereof and that the part of the absorbent core 4 that is located between the notches 4d on each side may separate apart from the diaper main body.

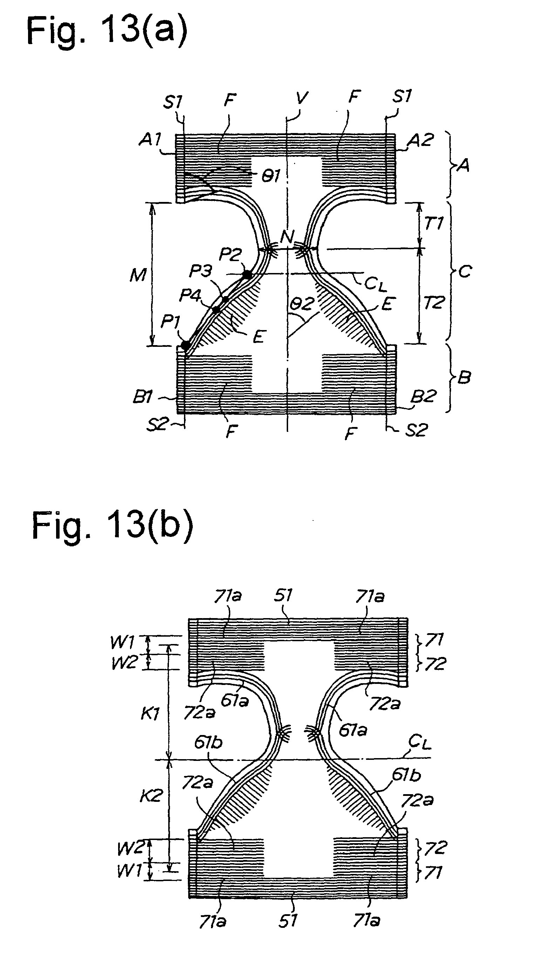

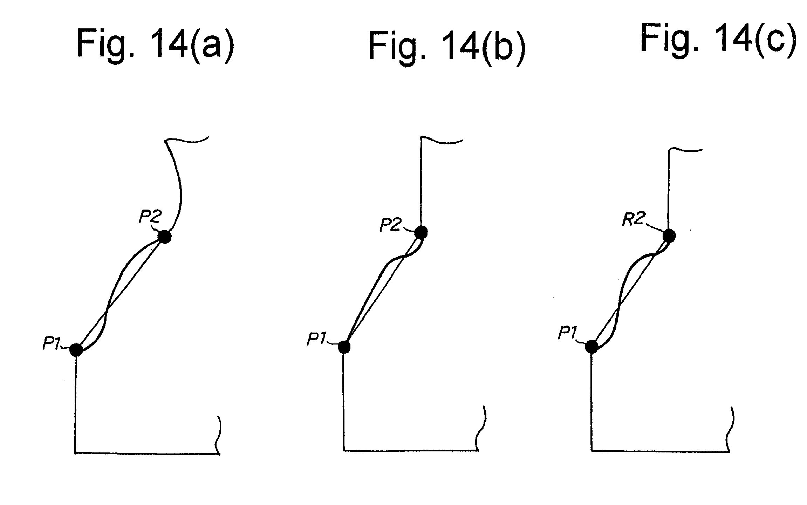

A further embodinent of the present invention will then be illustrated with reference to Figs. 13 and 14. The description given above with respect to the present invention is appropriately applied to those particulars of the embodiment that are not described hereunder. In Figs. 13 and 14, elements identified with the same reference characters or numerals as in Figs. 1 through 12 may be identical and will not be redundantly described.

In Figs. 13(a) and 13(b) is shown a plan of the exterior material used in a pull-on disposable diaper in its flat-out state. Figs. 13(a) and 13(b) represent the same plan so that reference numerals and characters may be allocated between them for the sake of easy reading. A perspective view of the diaper of the present embodiment and an exploded perspective view of the diaper before assembly are the same as those presented with regard to the pull-on disposable diaper according to the first aspect of the invention. Accordingly, Figs. 1 and 2 will be referred to with reference to the perspective view and the exploded perspective view of the diaper according to the second aspect. In the following description, "imaginary development view" is a view of a pull-on diaper imaginarily cut apart vertically exactly in the sides and opening the cut diaper flat, and "imaginary vertical cutting line" is a line along which a pull-on diaper is imaginarily cut apart vertically right in the side.

The diaper 1 of the present embodiment has side seals S exactly in the sides thereof. Therefore, the imaginary development view of the diaper 1 obtained by imaginarily cutting the diaper 1 vertically exactly in the sides and opening the diaper 1 flat has the same outline as a development view of the exterior material 11, and the imaginary vertical cutting line coincides with the line running vertically on the side seal S.

As depicted in Fig. 13(a), the angle θ1 is smaller than 90°. The angle θ1 is defined to be an angle made between (1) one of the imaginary vertical cutting lines in the front side (i.e., one of the vertical lines S1 running on the side seals) of the exterior material 11 and the leg hole intersecting with the line S1. To put it plainly, the curve of the leg hole in the stomach portion A of the diaper 1 is inwardly gouged or concave. The angle θ1 is preferably 50° to 85°. On the other hand, in the rear side of the development view of the exterior material 11, the leg hole is convex outward from straight line P1-P2 connecting position P1, i.e., the lower end of one of the vertical lines S2 running on the side seals and position P2, i.e., the intersection between the lateral centerline CL and the leg hole.

Thus, in the diaper 1 of this embodiment, the leg hole in the stomach portion A is inwardly concave, while the leg hole in the back portion C is convex outward. This configuration is the result of the above-described design method. The thus designed leg holes provide an extremely snug fit to the groins of a wearer. As a result, the diaper 1 looks neatly, particularly at the crotch portion C, while worn. Gapping around the wearer's legs is prevented, which allows for reduction of constrictive force of the elastic members disposed around the leg holes. The diaper 1 hardly slides down due to the wearer's movement, and the rear side of the leg hole hardly slides up to expose the buttock. To allow reducing the constrictive force around the leg holes and to prevent sliding down or up lead to reduction of interference with the wearer's movement.

The expression "the leg hole in the back portion C is convex outward" is intended to include not only the shape of Fig. 13(a) but shapes that are convex not wholly but partly, such as the shapes illustrated in Figs. 14(a), 14(b), and 14(c).

To secure a better fit of the leg hole to the wearer's groin, the leg hole in the rear side of the exterior material 11 is shaped such that the position on line P1-P2 which is farthest from the leg hole (indicated by P3 in Fig. 13 (a)) is nearer to the center of the development view of the exterior material 11 than the mid point of line P1-P2 (indicated by P4) as shown in Fig. 13(a). To put it another way, the top of the convex leg hole is nearer to the side of the crotch portion C than to the other side (nearer to the center of the imaginary development view than the mid point P4). By this design, the leg holes provide a better fit to the wearer's groins.

To secure a better fit of the leg hole to the wearer's groins, the distance between position P3 on line P1-P2 of the convex part (at which the distance between line P1-P2 and the leg hole is largest) and mid point P4 of line P1-P2 is set at 0 to 50 mm, preferably 0 to 30 mm, more preferably 0 to 20 mm, as shown in Fig. 13(a).

The balance between the stomach portion A and the back portion B in the diaper 1 is also important for a good fit of the leg holes to the wearer's groins. In detail, the ratio of the length T1 from the lower end of the vertical line S (running in the side seal in the front side in the development view of the exterior material 11) to the narrowest part in the development view to the length T2 from the lower end of the vertical line S2 (running in the side seal in the rear side of the development view of the exterior material 11) to the narrowest part in the development view, i.e., T1:T2 is 25:75 to 40:60 as shown in Fig. 13(a). The T1:T2 ratio is preferably 30:70 to 35:65. If the T1:T2 ratio is out of that range, the leg holes fail to fit the three dimensional contour of the groins, and it would be necessary to dispose an elastic member for exerting excessive constrictive force in the diaper in order to prevent gapping between the diaper and a wearer's body. The appearance of the diaper while worn is also deteriorated. Where the narrowest part is not identified by a single position but extends in the length direction with the same width, the position at the center of the length of the part with the same width is defined to be the narrowest part.

In order for the leg holes to maintain a proper fit to the wearer's groins even when the wearer moves, the present inventors have found it advantageous that the peripheral region of each leg hole in the rear side of the development view of the exterior material 11 is extensible in the direction perpendicular to that peripheral region. To accomplish this, it is preferred that elastic members E be arranged in the leg hole peripheral region of the rear side between the outer nonwoven fabric and the inner nonwoven fabric to impart the region with extensibility, as shown in Fig. 13(a). The elastic members E are preferably disposed to extend in a direction making an angle θ2 of 10° to 90°, more preferably 30° to 60°, with the longitudinal centerline V of the development view of the exterior material 11. By the provision of the elastic members E, the region is capable of flexibly extending and contracting with the skin extension and contraction accompanying the leg movement so that the region is prevented from getting baggy or sliding up to expose the buttock. The diaper 1 thus provides improved comfort and appearance while worn.

In order to further improve the appearance of the diaper during wear, the distance between the lower end of the vertical line S1 running in the side seal of the front side and the lower end of the vertical line S2 running in the side seal of the rear side of the exterior material 11 in its flat-out state shown in Fig. 13 (a), namely, the flap-to-flap distance M as explained with respect to the first aspect of the present invention is preferably 210 to 280 mm, more preferably 230 to 270 mm, even more preferably 230 to 260 mm.

In order that an absorbent core 4 of a proper amount (or a proper length or area) may be present along the crotch of a wearer so as to improve the appearance of the crotch portion C, the width at the narrowest part of the crotch portion C, i.e., the width N at the narrowest part in the longitudinal central portion of the exterior material 11 in
its flat-out state (see Fig. 13(a)) is preferably 50 to 150 mm, more preferably 60 to 140 mm, even more preferably 60 to 120 mm, similarly to the embodiment of the invention described above.

To prevent the diaper 1 from sliding down and further improve the appearance of the diaper 1 while worn, the pressure exerted by the previously identified first portion 71 of the diaper 1 is 1.1 to 2.5 kPa, preferably 1.1 to 2.0 kPa, more preferably 1.2 to 1.8 kPa, for the same reason as mentioned above. The first portion 71 is formed in the region of the diaper 1 that is applied to the upper iliac region of a wearer. The wearing pressure of the first portion 71 is measured by the method described above.

In the diaper 1 of the present embodiment, the width W 1 of the first portion 71 shown in Fig. 13(b) (i.e., the size of the first portion 71 measured in the longitudinal direction of the diaper 1) is 15 to 35 mm for the same reason as described above. The width W is more preferably 20 to 35 mm, even more preferably 25 to 30 mm, for further ensuring the effect on prevention of diaper's sliding down and for further improving the appearance of the diaper 1 while worn and the ease of diapering with the diaper 1.

As shown in Fig. 13(b), the first portion 71 can successfully be applied to the upper iliac region of a wearer when the distance K1 from the widthwise center of the first portion 71 (i.e., the center of the first portion 71 along the diaper length direction) of the stomach portion A to the lateral centerline CL of the diaper 1 is 180 to 220 mm as measured in the flat-out state of the diaper 1, and when the distance K2 from the widthwise center of the first portion 71 (i.e., the center of the first portion 71 along the diaper length direction) of the back portion B to the lateral centerline CL of the diaper 1 is 180 to 220 mm as measured in the flat-out state of the diaper 1, for the same reason as described above. Applying the first portion 71 to the wearer's upper iliac region will be achieved more successfully when the distances K1 and K2 are more preferably 185 to 215 mm, even more preferably 190 to 215 mm.

Similarly to the diaper of the embodiment described above, the diaper 1 of the present embodiment preferably relies for constriction primarily on the first elastic members 71a disposed in the first portion 71. From this viewpoint, the wearing pressure of the waist portion 5 of the diaper 1 is preferably 0.3 to 1.5 kPa, which range is lower than that of conventional pull-on diapers. It is preferred that the pressure of the waist portion 5 be lower than the pressure of the first portion 71. The wearing pressure by the waist portion 5 is more preferably 0.4 to 1.2 kPa, even more preferably 0.4 to 1.0 kPa, to prevent the diaper 1 from moving down more effectively.

Similarly to the diaper described above, the diaper 1 of the present embodiment preferably has a wearing pressure by the leg portion 6 of 1.0 to 2.4 kPa, more preferably 1.2 to 2.2 kPa, for the same reason as above. The wearing pressure by the leg portion is measured by the method previously described.

Similarly to the diaper described above, the diaper of the present embodiment preferably has a wearing pressure of 0.2 to 0.8 kPa, more preferably 0.3 to 0.6 kPa, in the area except the first portions 71, the waist portion 5, and the leg portions 6, for example, the second portions 72 between the first portions 71 and the leg portions 6 for the same reason as above. When the diaper 1 is worn, the second portions 72 are applied to the body below the upper iliac region, namely the lower abdominal region. The second portion 72 preferably has a width W2 (measured in the length direction of the diaper 1) of 40 to 70 mm, more preferably 45 to 65 mm, as shown in Fig. 13(b).

Comparing the wearing pressure between different portions of the diaper 1 of the present embodiment, it is preferred that the first portion 71 be the highest, the waist portion 5 be the next highest, and the second portion 72 be the lowest similarly to the diaper described above. The reason for the preference is the same as described above.

In the diaper according to the present embodiment, the portion of the absorbent core which is located in the wearer's crotch is preferably small in both the length and the width directions. In other words, the amount of the portion of the absorbent core located in the wearer's crotch is preferably reduced. However, this can lead to a reduced absorption capacity of the diaper. To avoid this, the diaper of the second aspect preferably uses an absorbent core having the configuration illustrated in Fig. 6 similarly to the diaper described above.

Where the absorbent core 4 of the configuration shown in Fig. 6 is used in the present embodiment, the substantial width of the absorbent core 4 during use (i.e., the width of the adhered area between the absorbent core 4 and the diaper main body) is preferably set at 20 to 90 mm. In view of fit and bagginess prevention in the crotch portion during use, that width is more preferably 20 to 80 mm, even more preferably 30 to 70 mm. The width of the absorbent core 4 before use (i.e., the total width of the absorbent core 4 before the side pieces separate) is preferably 50 to 150 mm. To secure the absorption performance of the crotch portion, that width is more preferably 60 to 150 mm, even more preferably 70 to 140 mm.

In the present embodiment, the absorbent core shown in Fig. 6 may be replaced with any of absorbent cores having the configurations illustrated in Figs. 10, 11, and 12(a) through 12(e).

While the present invention has been described with reference to its preferred embodiments, the present invention is not limited to these embodiments. For example, only one of the inner nonwoven sheet 13 and the outer nonwoven sheet 12 of the exterior material 11 may be formed of water repellent nonwoven fabric. Where the side cuffs 8 provide sufficient protection against leakage, it is possible to make both the inner nonwoven sheet 13 and the outer nonwoven sheet 14 of water permeable nonwoven fabric.

The diaper of the embodiment represented by Figs. 13(a) and 13(b) according to the invention has side seals S exactly in the sides thereof. Therefore, the imaginary development view of the diaper agrees with the development view of its exterior material 11. The invention is also applicable to a diaper the side seals of which are positioned slightly off the sides to the stomach side or the back side, for example, the diaper disclosed in JP-A-9-6607 cited above in Background Art.

### Examples

The present invention will now be illustrated in greater detail with reference to Examples, but it should be understood that the present invention is not construed as being limited thereto.

### EXAMPLES 1 AND 2 AND COMPARATIVE EXAMPLES 1 TO 3

Pull-on diapers shown in Figs. 1 through 3 were made. The absorbent body 10 was fabricated using hydrophilic air-through nonwoven fabric having a weight of 25 g/m² as a topsheet 2 and a polyethylene sheet having a weight of 20 g/m² as a backsheet 3. The exterior material 11 was fabricated using water repellent air-through nonwoven fabric having a weight of 25 g/m² as an outer nonwoven sheet 1 and water repellent spun-bonded nonwoven fabric having a weight of 18 g/m² as an inner nonwoven sheet. The wearing pressures exerted by elastic members in different portions were adjusted by changing the kind and the elongation of the elastic members. Measurements of the resulting diapers are shown in Table 1 below.

The resulting diapers were worn by 3 children of one to two years of age and evaluated by their mothers on the conditions of the diaper while worn according to the following rating systems. The results obtained are shown in Table 1.
(1) Bagginess of diaper around the crotch while the diaper is worn
   A: Bothering
   B: Slightly bothering
   C: Not bothering
(2) Ease for wearer to move while the diaper is worn
   A: No interference with the movement.
   B: Slight interference with the movement.
   C: Interference with the movement.
(3) Ease for mother to put a pair of pants on child with diaper
   A: Easy
   B: Slightly easy
   C: No easy
(4) Bagginess of pants around crotch on diaper
   A: Bothering
   B: Slightly bothering
   C: Not bothering
(5) Sliding down of diaper after 60 minutes wearing
   A: Not observed
   B: Slightly observed
   C: Observed

**TABLE 1**

| | Flap-to-Flap Distance (mm) | Width of Part Narrowest Part (mm) | Bagginess around Crotch | Ease of Movement | Ease of Putting Pants over Diaper | Bagginess of Pants around Pants around Crotch | Sliding Down of Diaper |
|---|---|---|---|---|---|---|---|
| Example 1 | 250 | 100 | A | A | A | A | A |
| Example 2 | 270 | 150 | A | A | A | A | A |
| Comp. Example 1 | 250 | 180 | B | B | A | C | B |
| Comp. Example 2 | 295 | 120 | C | B | B | C | C |
| Comp. Example 3 | 290 | 185 | C | C | C | C | C |

As is apparent from the results in Table 1, the diapers of Examples are free from bagginess around the crotch, making it easy for a wearer to move and for a carer to put a pair of pants thereover, provide the pants with a neat appearance without bagginess, and are maintained in right position during use. To the contrary, the diaper of Comparative Example 1, having a large width at the narrowest part of the crotch portion, causes the crotch portion of itself and of the pants put thereover to become baggy, interferes with the body movement, and easily slides down. The diaper of Comparative Example 2, having a large flap-to-flap distance, becomes baggier than the diaper of Comparative Example 1 in its crotch portion and causes more bagginess in the crotch portion of the pants put thereon. Having a large width at the narrowest part of the crotch portion and a large flap-to-flap distance, the diaper of Comparative Example 3 is inferior in every aspect of the evaluation.

### EXAMPLES 3 AND 4 AND COMPARATIVE EXAMPLES 4 TO 7

Pull-on diapers shown in Figs. 1, 2, 13(a), and 13(b) were made. The absorbent body 10 was fabricated using hydrophilic air-through nonwoven fabric having a weight of 25 g/m² as a topsheet 2 and a polyethylene sheet having a weight of 20 g/m² as a backsheet 3. The exterior material 11 was fabricated using water repellent air-through nonwoven fabric having a weight of 25 g/m² as an outer nonwoven sheet 11 and water repellent spun-bonded nonwoven fabric having a weight of 18 g/m² as an inner nonwoven sheet. Measurements of the resulting diapers are shown in Table 2 below.

The resulting diapers were worn by 3 children of one to two years of age and evaluated for the following. The results obtained are shown in Table 2.
(1) Elastic mark left around the legs after 60 minute wearing
   A: No mark
   B: Slightly visible mark
   C: Visible mark
(2) Position of the waist after 60 minutes wearing
   A: Unchanged
   B: Slightly changed
   C: Changed
(3) Position of rear leg hole after 60 minutes wearing
   A: Unchanged
   B: Slightly changed (slight slid up)
   C: Changed (slid up)

**TABLE 2**

| | θ1 (°) | Concave or Convex from P1-P2 | Position on P1-P2 Farthest from Leg Hole | P3-P4 (mm) | T1:T2 | Elastic Marks on Legs | Sliding down from Waist | Sliding up from Rear Leg Holes |
|---|---|---|---|---|---|---|---|---|
| Example 3 | 75 | convex | nearer to the center than mid point | 9.6 | 33:67 | A | A | A |
| Example 4 | 82 | convex | nearer to the center than mid point | 25.3 | 39:61 | A | A | A |
| Comp. Example 4 | 100 | convex | nearer to the center than mid point | 9.6 | 33:67 | C | B | A |
| Comp. Example 5 | 75 | non-convex | - | - | 35:65 | A | A | C |
| Comp. Example 6 | 74 | convex | farther from the center than mid point | - | 34:66 | A | A | C |
| Comp. Example 7 | 75 | convex | nearer to the center than mid point | 56.3 | 46:54 | B | C | C |

It is apparently seen from the results in Table 2 that the diapers of Examples are prevented from sliding down at the waist or sliding up on the rear leg holes without giving excessive constriction around the legs. To the contrary, the diapers of Comparative Examples 4 to 7, in which the shape of the leg holes is out of the range specified in the present invention, is too constrictive around the legs (Comparative Example 4) or suffer from sliding down at the waist and/or sliding up in the rear leg holes (Comparative Examples 5 to 7).

### Industrial Applicability

The pull-on disposable diaper according to the present invention has reduced bagginess around the crotch portion to give a neat appearance as a whole diaper while worn. The wearer's movement, particularly the leg movement is not hindered. The diaper hardly slides down with the wearer's movement while worn so that urine and fecal leakage hardly occurs. Outer clothing such as a shorts or a pants can easily be put over the diaper to give a neat appearance.

The pull-on disposable diaper according to the present invention has the crotch portion thereof designed to snugly fit the wearer's groins to give the crotch portion a neat appearance. Where an elastic member is disposed around the leg holes to prevent gapping around the wearer's legs, the invention allows for reduction of the constrictive force of the elastic member. The diaper hardly slides down at the waist or slides up at the rear leg holes, which would make the buttocks exposed, while worn due to the wearer's movement. In particular, the diaper hardly becomes baggy around the crotch. To allow reducing the constrictive force around the leg holes and to prevent sliding down or up lead to reduction of interference with the wearer's movement.

## Claims

1. A pull-on disposable diaper having a waist opening (5) and a pair of leg openings (6),
the diaper (1) having, in a flat-out state thereof, a distance (M) between the lower end of a flap (F) in the longitudinally front portion and the lower end of a flap (F) in the longitudinally rear portion of 210 to 280 mm and a width (N) of the narrowest part located near the longitudinal center of 50 to 160 mm,
the diaper having a portion (71) exerting a pressure of 1.1 to 2.5 kPa while the diaper is worn, the portion (71) being located between the waist opening (5) and the leg opening (6) in each of a stomach portion (A) and a back portion (B) of the diaper, and having a width W1, measured in the diaper length direction, of 15 to 35 mm, wherein the pressure is exerted by elastic members (71a) being fixed in their stretched state, wherein the portion (71) exists in a region adapted to be applied to the part of a wearer from the iliac crest to the anterior superior iliac spine, wherein measurement of the wearing pressure of the portion (71) of the diaper (1) is carried out on the diaper (1) put on a cylinder having a circumference of 500 mm with a clothing pressure measuring device of the air-pack type contact surface pressure measuring system AMI 3037-2, available from AMI Techno Co., Ltd.

2. The pull-on disposable diaper according to claim 1, wherein an absorbent core (4) is configured to separate apart from a main body of the diaper (1) in side parts located near the longitudinal side edges thereof and has a part which is located between the side parts and is fixed to the main body.

3. The pull-on disposable diaper according to claim 1 or 2, wherein, in the flat-out state of the diaper, the distance from the lateral centerline (CL) of the diaper (1) to the center of the portion (71) of the stomach portion (A) is 180 to 220 mm, and the distance from the lateral centerline (CL) of the diaper to the center of the portion (71) of the back portion (B) is 180 to 220 mm.

4. The pull-on disposable diaper according to claim 1, 2 or 3, wherein the portion (71) is located between both sides of the diaper (1) and both longitudinal sides of an absorbent body.

5. The pull-on disposable diaper according to any one of claims 1 to 4, wherein a waist portion (1) of the diaper while worn exerts a pressure of 0.3 to 1.5 kPa, the measurement of the wearing pressure of the waist portion (1) being carried out as defined in claim 1, wherein the pressure is exerted by elastic members fixed in their stretched state.

6. The pull-on disposable diaper according to any one of claims 1 to 5, wherein the elastic members extend in the width direction of the diaper.

7. The pull-on disposable diaper according to any one of claims 1 to 5, wherein the diaper further comprises an exterior material having at least two sheets of nonwoven fabric, and the elastic members are fixed between the sheets.

## Patentansprüche

1. Einweg-Höschenwindel mit einer Taillenöffnung (5) und einem Paar Beinöffnungen (6), wobei die Windel (1) in ihrem ausgebreiteten Zustand einen Abstand (M) zwischen dem unteren Ende einer Klappe (F) in dem in Längsrichtung vorderen Abschnitt und dem unteren Ende einer Klappe (F) in dem in Längsrichtung hinteren Abschnitt von 210 bis 280 mm und eine Breite (N) in dem schmalsten Teil, der sich nahe der Längsmitte befindet, von 50 bis 160 mm hat,
wobei die Windel einen Abschnitt (71) hat, der einen Druck von 1,1 bis 2,5 kPa ausübt, während die Windel getragen wird, wobei der Abschnitt (71) sich jeweils in einem Bauchabschnitt (A) und einem Rückenabschnitt (B) der Windel zwischen der Taillenöffnung (5) und der Beinöffnung (6) befindet und eine in der Windellängsrichtung gemessene Breite W1 von 15 bis 35 mm hat, wobei der Druck von elastischen Elementen (71a) ausgeübt wird, die in ihrem gedehnten Zustand befestigt sind, wobei der Abschnitt (71) in einem Bereich vorhanden ist, der geeignet ist, auf den Teil eines Trägers von dem Beckenkamm zu der vorderen oberen Beckenwirbelsäule angelegt zu werden, wobei die Messung des Tragedrucks des Abschnitts (71) der Windel (1) an der Windel (1), die auf einen Zylinder mit einem Umfang von 500 mm aufgebacht ist, mit einer Kleidungsdruckmessvorrichtung vom Luftverdichtungs-Oberflächenkontaktdruckmesssystem AMI 3037-2, erhältlich von der AMI Techno Co., Ltd., durchgeführt wird.

2. Einweg-Höschenwindel nach Anspruch 1, wobei ein absorbierender Kern (4) aufgebaut ist, so dass er von einem Hauptkörper der Windel (1) in Seitenteilen, die sich nahe ihren Längsseitenrändern befinden, getrennt ist, und der einen Teil hat, der sich zwischen den Seitenteilen befindet und an dem Hauptkörper befestigt ist.

3. Einweg-Höschenwindel nach Anspruch 1 oder 2, wobei der Abstand von der seitlichen Mittellinie (CL) der Windel (1) zu der Mitte des Abschnitts (71) des Bauchteils (A) in dem ausgebreiteten Zustand 180 bis 220 mm ist, und der Abstand von der seitlichen Mittellinie (CL) der Windel zu der Mitte des Abschnitts (71) des Rückenabschnitts (B) 180 bis 220 mm ist.

4. Einweg-Höschenwindel nach Anspruch 1, 2 oder 3, wobei der Abschnitt (71) sich zwischen beiden Seiten der Windel (1) und beiden Längsseiten eines absorbierenden Körpers befindet.

5. Einweg-Höschenwindel nach einem der Ansprüche 1 bis 4, wobei ein Taillenabschnitt (1) der Windel, während sie getragen wird, einen Druck von 0,3 bis 1,5 kPa ausübt, wobei die Messung des Tragedrucks des Taillenabschnitts (1), wie in Anspruch 1 definiert, ausgeführt wird, wobei der Druck von elastischen Elementen ausgeübt wird, die in ihrem gedehnten Zustand befestigt sind.

6. Einweg-Höschenwindel nach einem der Ansprüche 1 bis 5, wobei die elastischen Elemente sich in der Breitenrichtung der Windel erstrecken.

7. Einweg-Höschenwindel nach einem der Ansprüche 1 bis 5, wobei die Windel ferner ein Außenmaterial mit wenigstens zwei Schichten aus Vliesgewebe aufweist und die elastischen Elemente zwischen den Schichten befestigt sind.

## Revendications

1. Couche jetable de type culotte comportant une ouverture pour taille (5) et une paire d'ouvertures pour jambe (6), la couche (1) ayant, dans son état aplati, une distance (M) entre l'extrémité inférieure d'un rabat (F) dans la portion longitudinalement avant et l'extrémité arrière d'un rabat (F) dans la portion longitudinalement arrière de 210 à 280 mm et une largeur (N) de la partie la plus étroite située près du centre longitudinal de 50 à 160 nm,
la couche ayant une portion (71) exerçant une pression de 1,1 à 2,5 kPa quand la couche est portée, la portion (71) étant située entre l'ouverture pour taille (5) et l'ouverture pour jambe (6) dans chacune d'une portion de ventre (A) d'une portion de dos (B) de la couche, et ayant une largeur W1, mesurée dans la direction de la longueur de la couche, de 15 à 35 mm, où la pression est exercée par des organes élastiques (71a) fixés dans leur état étiré, où la portion (71) existe dans une région adaptée pour être appliquée à la partie d'un porteur de la crête iliaque à l'épine iliaque antéro-supérieure, où une mesure de la pression de port de la portion (71) de la couche (1) est réalisée sur la couche (1) placée sur un cylindre ayant une circonférence de 500 mm avec un dispositif de mesure de pression de vêtement du système de mesure de pression de surface par contact de type matelas gonflable AMI 3037-2, disponible auprès de AMI Techno Co., Ltd.

2. Couche jetable de type culotte selon la revendication 1, dans laquelle un coeur absorbant (4) est configuré pour se séparer d'un corps principal de la couche (1) dans les parties latérales situées près des bords latéraux longitudinaux de celle-ci et comporte une partie qui est située entre les parties latérales et est fixée au corps principal.

3. Couche jetable de type culotte selon la revendication 1 ou 2, dans laquelle, dans l'état aplati de la couche, la distance de la médiane latérale (CL) de la couche (1) au centre de la portion (71) de la portion de ventre (A) est de 180 à 220 mm, et la distance de la médiane latérale (CL) de la couche au centre de la portion (71) de la portion de dos (B) est de 180 à 220 mm.

4. Couche jetable de type culotte selon la revendication 1, 2 ou 3, dans laquelle la portion (71) est située entre les deux côtés de la couche (1) et les deux côtés longitudinaux d'un corps absorbant.

5. Couche jetable de type culotte selon l'une quelconque des revendications 1 à 4, dans laquelle une portion de taille (1) de la couche lorsqu'elle est portée exerce une pression de 0,3 à 1,5 kPa, la mesure de la pression de port de la portion de taille (1) étant réalisée telle que définie dans la revendication 1, où la pression est exercée par des organes élastiques fixés dans leur état étiré.

6. Couche jetable de type culotte selon l'une quelconque des revendications 1 à 5, dans laquelle les organes élastiques s'étendent dans la direction de la largeur de la couche.

7. Couche jetable de type culotte selon l'une quelconque des revendications 1 à 5, dans laquelle la couche comprend en outre un matériau extérieur ayant au moins deux feuilles d'étoffe non tissée, et les organes élastiques sont fixés entre les feuilles.
